# EUROPÄISCHE PATENTSCHRIFT

(11) **EP 3 295 803 B1**
(45) Veröffentlichungstag und Bekanntmachung des Hinweises auf die Patenterteilung: **24.02.2021**
(21) Anmeldenummer: 17197218.5
(22) Anmeldetag: 26.02.2010
(51) Int. Cl.: A23J 1/14, A61K 8/64, A23K 10/30, A23L 33/185, A61Q 19/00, A23K 20/147, A23K 50/80

(54) **PROTEINPRÄPARATE AUS SONNENBLUMENSAMEN UND DEREN HERSTELLUNG**
PROTEIN-PREPARATIONS FROM SUNFLOWER SEEDS AND THEIR MANUFACTURE
PREPARATIONS DE PROTEINES DE GRAINES DE TOURNESOL ET LEUR PROCEDE DE PREPARATION

(30) Priorität: 27.02.2009 DE 102009010813
(43) Veröffentlichungstag der Anmeldung: 21.03.2018
(62) Teilanmeldung aus: 10707826.3
(73) Patentinhaber: Fraunhofer-Gesellschaft zur Förderung der angewandten Forschung e.V., 80686 München (DE)
(72) Erfinder: Pickardt, Claudia, 12203 Berlin (DE); Eisner, Peter, 85354 Freising (DE); Bader, Stephanie, 85368 Moosburg (DE); Wild, Florian, 85354 Freising (DE); Müller, Klaus, 85356 Freising (DE)
(74) Vertreter: Gagel, Roland

(56) Entgegenhaltungen:
- DD-A1- 269 086
- DE-A1- 2 545 043
- BOURGES R H ET AL: "Obtencion de harina y de un concentrado proteinico a partir de semillas de heliantus annus y su incorporacion en galletas", ARCHIVOS LATINOAMERICANOS DE NUTRICION, Bd. 30, Nr. 4, 1980, Seiten 564-579, XP009135815,
- Lin M J Y: "Certain Functional Properties of Sunflower Meal Products", Journal of Food Science , Bd. 39, Nr. 2 1974, 1974, Seiten 368-370, XP002590913, ISSN: 0022-1147 Gefunden im Internet: URL:http://www3.interscience.wiley.com/cgi -bin/fulltext/119662970/PDFSTART?CRETRY=1& SRETRY=0 [gefunden am 2010-07-08]
- SAEED M ET AL: "Sunflower protein concentrates and isolates low in polyphenols and phytate.", JOURNAL OF FOOD SCIENCE, Bd. 53, Nr. 4, Juli 1988 (1988-07), Seiten 1127-1131, XP002590909, DOI: 10.1111/j.1365-2621.1988.tb13545.x
- MILLER N; PRETORIUS H E; TOIT L J DU: "Phytic acid in sunflower seeds, pressed cake and protein concentrate", FOOD CHEMISTRY, Bd. 21, Nr. 3, 1986, Seiten 205-209, XP002776187, WNNR, Nasionale Voedselnavorsinginst., PO Box 395, Pretoria 0001, South Africa DOI: 10.1016/0308-8146(86)90018-X
- GASSMANN B: "Preparation and application of vegetable proteins, especially proteins from sunflower seed, for human consumption. An approach.", NAHRUNG, Bd. 27, Nr. 4, 1983, Seiten 351-369, XP002590910, DOI: 10.1002/food.19830270408
- TRANCHINO L ET AL: "Almost complete dehulling of high oil sunflower seed.", JOURNAL OF THE AMERICAN OIL CHEMISTS' SOCIETY, Bd. 61, Nr. 7, Juli 1984 (1984-07), Seiten 1261-1265, XP002590911, DOI: 10.1007/BF02636267
- TSUNG MIN KUO ET AL: "Content of raffinose oligosaccharides and sucrose in various plant", JOURNAL OF AGRICULTURAL AND FOOD CHEMISTRY, AMERICAN CHEMICAL SOCIETY, BOOKS AND JOURNALS DIVISION, Bd. 36, Nr. 1, 1988, Seiten 32-36, XP002193475, ISSN: 0021-8561, DOI: 10.1021/JF00079A008
- MIETH G ET AL: "Sunflower seeds and products of their processing. I. Production and value-determining constituents. (translated) TIOL- Sonnenblumensamen und deren Verarbeitungsprodukte. I. Aufkommen und wertbestimmende Bestandteile.", DIE NAHRUNG, Bd. 28, Nr. 5, 1984, Seiten 533-577, XP002590914, DOI: 10.1002/FOOD.19840280509
- Claude Balny ET AL: "Effects of high pressure on proteins", FOOD REVIEWS INTERNATIONAL, vol. 9, no. 4, 1 November 1993 (1993-11-01), pages 611-628, XP055713072, Philadelphia, USA ISSN: 8755-9129, DOI: 10.1080/87559129309540980
- W. Messens ET AL: "The use of high pressure to modify the functionality of food proteins", Trends in Food Science & Technology, vol. 8, no. 4, 1 April 1997 (1997-04-01), pages 107-112, XP055084041, ISSN: 0924-2244, DOI: 10.1016/S0924-2244(97)01015-7

## Beschreibung

### Technisches Anwendungsgebiet

Die vorliegende Erfindung betrifft ein Proteinpräparat aus Sonnenblumensamen mit verbesserten Anwendungseigenschaften.

### Stand der Technik

Proteinpräparate finden vielfach Anwendung in Lebensmitteln als ernährungsphysiologisch oder technofunktionell aktive Zutaten. Es gibt Proteinpräparate mit besonders hoher Proteinwertigkeit zur Anwendung als hochwertige Nahrungsmittelzusätze (Babynahrung, Spezialernährung, Sportlernahrung). Diese sind prinzipiell auch für die Formulierung von Futtermitteln von Interesse, in denen eine hohe Proteinverfügbarkeit gewährleistet werden muss. Andere Proteinpräparate haben eine gute Technofunktionalität und sind z.B. dafür geeignet, Schäume oder Emulsionen zu stabilisieren oder Gele auszubilden. Diese Proteinpräparate sind vorrangig als Lebensmittelzutaten geeignet und finden außerdem Anwendung für Spezial-Futtermittel oder technische Zwecke.

Grundsätzlich können Proteinpräparate tierischen und pflanzlichen Ursprungs unterschieden werden. Beispiele von Proteinpräparaten tierischen Ursprungs sind solche aus Hühnerei, Milch, Molke oder Kasein und Gelatinepräparate aus Schlachtabfällen. Nachteilig ist, dass solche Proteinpräparate einen charakteristischen Eigengeschmack und Eigengeruch aufweisen und daher auf bestimmte Anwendungen beschränkt sind. Oft sind sie teuer in der Herstellung und problematisch in Bezug auf Allergien und werden von bestimmten Verbrauchern aus ethischen Gründen abgelehnt.

Man unterscheidet bei Pflanzenproteinpräparaten aufgrund der Herstellung und des sich daraus ergebenden Proteingehalts zwischen Proteinkonzentraten und Proteinisolaten. Im Vergleich zu Pflanzenproteinkonzentraten mit einem Proteingehalt zwischen 60 % und 90 % haben Proteinisolate einen sehr hohen Proteingehalt, der mindestens 90 % beträgt. Zur Herstellung von Proteinisolaten werden die Proteine in Wasser gelöst und anschließend aus der wässrigen Lösung isoliert. Sie haben daher gegenüber den extrahierten Pflanzensamen ein verändertes Aminosäureprofil und veränderte Nährwert- und technofunktionelle Eigenschaften.

Als pflanzliche Proteinpräparate sind am Markt hauptsächlich Sojaproteinpräparate, nämlich Sojaproteinkonzentrate und -isolate, und Weizenkleberpräparate verfügbar. Daneben werden Proteinpräparate aus anderen Leguminosenproteinen, wie z.B. Erbsenproteinkonzentrate, angeboten.

Bekannt sind ebenfalls pflanzliche Proteinpräparate, Proteinkonzentrate und Proteinisolate, aus entölten Ölsamen, wie z.B. Rapssamen und Sonnenblumenkernen. Diese werden jedoch zurzeit fast ausschließlich zur Ölgewinnung genutzt. Die dabei anfallenden Press- und Extraktionsrückstände (Presskuchen und Schrote) werden bislang trotz des hohen ernährungsphysiologischen und technofunktionellen Potenzials im Gegensatz zu Soja nicht im Lebensmittelbereich verwertet. Ein Grund hierfür ist der Anteil störender Begleitstoffe, wie Polyphenole, die den Geschmack und die Farbe der Produkte beeinträchtigt.

Ölsamen und Leguminosen werden nach Stand der Technik mit Hexan entölt. Leguminosensamen werden dazu geschält, flockiert und in einer Extraktionsanlage mit Hexan extrahiert. Ölsamen werden alternativ flockiert und direkt entölt oder mechanisch teilentölt (Vorpressung) und anschließend durch Extraktion fertig entölt, wobei der Presskuchen vor der Extraktion aufgebrochen werden muss, um die Extraktion zu ermöglichen. Auch eine Fertigpressung bis auf Restölgehalte von ca. 5% ohne nachfolgende Extraktion wird durchgeführt, wobei der Restölgehalt in den Expellern (Presskuchen, Schilfern) die Lagerstabilität senkt.

Sonnenblumenkerne werden bisher überwiegend ungeschält oder nur zu maximal 2/3 geschält zur Entölung eingesetzt. Insbesondere zur Pressung, d.h. Fertigpressung oder Vorpressung als Teilentölung, wird ein hoher Schalenanteil als notwendig erachtet. Die Presskuchen und Schrote sind in diesen Fällen dunkel gefärbt und haben einen sehr hohen Rohfasergehalt. Sie eignen sich daher nicht zur Herstellung von hochwertigen Proteinmehlen und -konzentraten.

Es gibt unterschiedliche Ansätze, Proteine aus den Rückständen der Sonnenblumenölgewinnung zu isolieren. Die Entfernung von störenden Polyphenolen, hauptsächlich Chlorogensäure, die die Farbe von Sonnenblumen-Proteinisolaten beeinträchtigt, steht dabei im Vordergrund. Hierfür wurden bislang Extraktionen mit unterschiedlichen Lösungsmitteln, darunter auch Wasser und Alkohole zur Entfernung der Polyphenole aus entölten Sonnenblumenschroten vorgeschlagen. Die Gewinnung von Proteinisolaten aus Sonnenblumenkernen und -presskuchen oder -schroten ist besonders schwierig wegen der geringen Wasserlöslichkeit der Sonnenblumenproteine, die den Einsatz von Alkali oder Salzen erfordert. Dies zieht einen besonders hohen Wasserverbrauch zur Proteinaufbereitung (Waschen) nach sich, verbunden mit hohen Proteinverlusten, was die Herstellungskosten der Proteinisolate erhöht und damit ihr Applikationsspektrum einschränkt.

Zur Entfernung der farbaktiven phenolischen Substanzen aus entfetteten Sonnenblumenkernen mit dem Ziel der anschließenden Proteinextraktion und Gewinnung von Proteinisolaten aus dem so vorbehandelten Material wurden unterschiedliche wässrige alkoholische Mischungen getestet, insbesondere Butanol in unterschiedlichen Anteilen mit salzsaurem Wasser, Ethanol mit einem Anteil von 95 % (v/v), Isopropanol (70 %, v/v) und Methanol (80 % v/v). Nachteilig bei der Extraktion mit diesen Lösemitteln ist die weitgehende Denaturierung der Proteine durch die Lösemittelbehandlung, so dass die Proteinlöslichkeit stark herabgesetzt wird. Dadurch sind die nachfolgende Extraktion von Proteinen bei der Herstellung von Proteinisolaten sowie deren Funktionalität stark eingeschränkt.

In der WO02/060273A1 wird ein Verfahren beschrieben, mit dem Proteinisolate mit einem Proteingehalt von mehr als 90% aus Sonnenblumensamen gewonnen werden. Die Proteine werden dafür wässrig extrahiert und durch eine Fällung mit Alkohol bei niedrigen Temperaturen gewonnen. Diese sind aufgrund des hohen Energieaufwandes für die Kühlung teuer und daher in ihrer Anwendung begrenzt.

Proteinkonzentrate aus Sonnenblumenkernen werden durch trocken- oder nasstechnische Aufbereitung gewonnen, wobei das Protein im Rückstand verbleibt. Der hohe Anteil an unerwünschten Begleitstoffen schränkt ihre Verwendung im Lebensmittelbereich ein. Insgesamt sind die bekannten Pflanzenproteinkonzentrate, welche einen niedrigen Aufreinigungsgrad aufweisen, eingeschränkt in ihrer Funktionalität und/oder enthalten einen gewissen Anteil störender Komponenten, die den Nährwert, die Farbe, den Geruch und/oder Geschmack der sie enthaltenden Lebens- oder Nahrungsmittel sehr negativ beeinflussen können. Proteinkonzentrate aus Sonnenblumenkernen haben daher eine beschränkte Applikationsbreite und sind nur in niedrigen Konzentrationen einsetzbar.

Lin M. J. Y. et al.: "Certain Functional Properties of Sunflower Meal Products", Journal of Food Science, Bd. 39, Nr. 2, 1974, Seiten 368-370, beschreiben ein Proteinmehl aus Sonnenblumen, für das die Sonnenblumensamen zunächst fein vermahlen werden, um sie anschließend mit Lösemittel auf den niedrigen Ölgehalt entölen zu können. Das damit erhaltenen Proteinmehl hat einen grünlichen oder bräunlichen Farbton. Zur Herstellung von Proteinkonzentraten mit einem helleren Farbton wird das entölte Sonnenblumenmehl nochmals mit wässrigem HCl behandelt. Dies führt allerdings zu einer niedrigen Proteinlöslichkeit.

Saeed M. et al.: "Sunflower protein concentrates and isolates low in polyphenols and phytate", Journal of Food Science, Bd. 53, Nr. 4, 1988, Seiten 1127-1131, beschreiben ein Proteinpräparat aus Sonnenblumensamen, für das die geschälten Sonnenblumensamen vor der Entölung mit einem Lösemittel zunächst fein vermahlen werden, um bei der Entölung einen niedrigen Ölgehalt im Bereich von 1% zu erreichen. Die feine Vermahlung hat jedoch Nachteile hinsichtlich der Temperaturbelastung und der Farbe der späteren Proteinpräparate.

In der DD 269 086 A1 wird ein Verfahren zur Herstellung von Proteinpräparaten aus Sonnenblumensamen beschrieben, bei dem das aus geschälten Sonnenblumensamen nach einer Vorpressung und Desolventierung erhaltene Mehl einer Temperaturbehandlung unterzogen wird. Die damit erhaltenen Proteinpräparate mit einem Proteingehalt von weniger als 90% weisen jedoch keine Helligkeit mit einem Helligkeitswert L von ≥ 70 und zudem auch eine relativ niedrige Emulgierkapazität auf.

Die Aufgabe der vorliegenden Erfindung besteht darin, ein Proteinpräparat bereitzustellen, das sensorisch ansprechend und vielseitig einsetzbar ist.

### Darstellung der Erfindung

Diese Aufgabe wird mit dem Proteinpräparat nach Anspruch 1 gelöst. Die weiteren Ansprüche geben bevorzugte Ausgestaltungen des Proteinpräparates an.

Bei dem in dieser Patentanmeldung nicht beanspruchten Verfahren zur Gewinnung des erfindungsgemäßen Proteinpräparates aus Sonnenblumensamen werden wenigstens folgende Schritte durchgeführt:
- Schälen der Sonnenblumensamen bis auf einen Restschalengehalt von ≤ 5 Gew.% oder Bereitstellen von geschälten Sonnenblumensamen mit einem Restschalengehalt von ≤ 5 Gew.% (jeweils bezogen auf die Gesamtmasse der Kernfraktion, wie sie unmittelbar nach dem Schälen erhalten wird);
- mechanische Teilentölung der geschälten Sonnenblumensamen durch Pressen bis auf einen Fett- oder Ölgehalt der geschälten Sonnenblumensamen im Bereich zwischen 10 und 35 Gew.%; und
- Durchführung eines oder mehrerer Extraktionsschritte mit mindestens einem Lösungsmittel, durch die ein entfettetes proteinhaltiges Mehl als Proteinpräparat erhalten wird. Mindestens einer der Extraktionsschritte wird bei dem Verfahren so ausgeführt, dass eine weitere Entölung der teilentölten geschälten Sonnenblumensamen bewirkt wird.

Durch die Kombination des geringen Restschalengehaltes und der mechanischen Teilentölung bis auf den angegebenen Restölgehalt können Proteinkonzentrate erhalten werden, die sowohl optisch als auch funktionell für einen Einsatz im Lebens- oder Futtermittelbereich sehr vorteilhafte Eigenschaften aufweisen. Das Verfahren ermöglicht eine besonders schonende Behandlung der Proteine, indem bei der mechanischen und/oder weiteren Entölung eine zu hohe Temperatur vermieden wird, die zu unerwünschten Proteinveränderungen und Aromaveränderungen führen könnte.

Die mechanische Teilentölung der Sonnenblumenkerne auf die angegebenen Restölgehalte wird vorzugsweise so durchgeführt, dass ein mechanisch stabiler Presskuchen mit einer Dicke im Bereich zwischen 0,2 und 4 cm, vorzugsweise im Bereich zwischen 0,5 und 2 cm, erhalten wird. Dadurch werden die anschließenden Verfahrensschritte vereinfacht, da aufgrund der Porosität und Dicke des Presskuchens auf eine mechanische Zerkleinerung vor der weiteren Extraktion verzichtet werden kann.

Das Verfahren erlaubt auch eine schonende Herstellung des Präparates in der Art, dass eine Denaturierung der Proteine in definierter Weise zugelassen wird. Die Teilentölung und der eine oder die mehreren Extraktionsschritte werden dabei so durchgeführt, dass der Denaturierungsgrad der Proteine im entfetteten proteinhaltigen Mehl (bezogen auf das Eingangsprodukt des Verfahrens) maximal 40%, vorzugsweise zwischen 10% und 30%, beträgt. Dies ermöglicht es, qualitativ und sensorisch hochwertige Proteinpräparate mit einem breiten Applikationsspektrum zu gewinnen.

Vorzugsweise wird die Extraktion mit einem Lösungsmittel oder Lösungsmittelgemisch in mehreren Extraktionsschritten durchgeführt, die eine Kombination von mindestens einem lipophilen Extraktionsschritt mit einem lipophilen Lösungsmittel bzw. Lösungsmittelgemisch und mindestens einem hydrophilen Extraktionsschritt mit einem hydrophilen Lösungsmittel bzw. Lösungsmittelgemisch umfassen. Weiterhin wird vorzugsweise die Konzentration des Extraktionslösungsmittels im letzten Extraktionsschritt soweit erhöht, dass eine anschließende Trocknung besonders einfach und schonend gestaltet werden kann.

Das mit dem Verfahren herstellbare Proteinpräparat aus Sonnenblumenkernen weist einen Proteingehalt von mindestens 50% auf. Es ist für eine kostengünstige Herstellung zugänglich, da eine hohe Aufreinigung, wie sie bei den Proteinisolaten erforderlich ist, vermieden werden kann.

Überraschenderweise weist das Proteinpräparat trotz des höheren Anteils an proteinfremden Stoffen Eigenschaften auf, die ähnlich wie die bekannten Proteinisolate aus diesen Rohstoffen oder sogar vielfältiger als diese sind. Aufgrund der hellen Farbe sowie des ausgewogenen technofunktionellen Spektrums in Form der wasserbindenden, ölbindenden sowie emulgierenden Funktionalität ist das Proteinpräparat vielseitig einsetzbar, u. a. in Lebens- und Futtermitteln, um Wasser und/oder Öl zu binden und/oder eine Emulsion zu bilden. Das Proteinpräparat ist geeignet, andere Präparate zu ersetzen, welche für diese Funktionalitäten bis anhin verwendet wurden und welche tierischen oder pflanzlichen Ursprungs sind wie Hühnerei, Milch, Soja in Form von Sojaproteinisolaten, etc.

Bereits in Form des besonders kostengünstig herstellbaren Sonnenblumenkernmehls, d.h. des aus dem Verfahren direkt erhaltenen, entfetteten proteinhaltigen Mehls, weist das Proteinpräparat überraschenderweise Eigenschaften hinsichtlich Farbe und Funktionalitäten auf, die es erlauben, das Proteinmehl direkt in zahlreichen Lebensmittel- und Futtermittelanwendungen einzusetzen.

Der Anwendungsbereich des Proteinpräparats kann noch weiter erstreckt werden, wenn das Proteinpräparat frei von den pflanzen- oder saateigenen Aromen ist, insbesondere wenn es im Wesentlichen geruchsfrei und/oder im Wesentlichen geschmacksneutral ist. Dadurch wird u. a. vermieden, dass es bei der Einarbeitung des Proteinpräparats in Lebens- und Futtermitteln zu einer unerwünschten Geschmacks- und Aromaänderung kommt.

Auch durch das Erhalten einer schaumbildenden Funktionalität kann der Anwendungsbereich erweitert werden, so dass das Proteinpräparat z. B. als Ersatz für Hühnereiklar oder andere schaumbildende Zusätze verwendet werden kann, um schaumartige Lebensmittel herzustellen.

Vorzugsweise weist das Proteinpräparat einen niedrigen Fettgehalt auf, wodurch eine gute Lagerstabilität des Proteinpräparats gewährleistet wird.

Weiter vorzugsweise weist das Proteinpräparat einen niedrigen Gehalt an Phytinsäure, Oligosacchariden und/oder Phenolsäuren auf. Dadurch ist der Gehalt an Stoffen reduziert, die bei der Verdauung die Verwertung von Nährstoffen beeinträchtigen können.

### Kurze Beschreibung der Zeichnungen

Im Folgenden werden das Herstellungsverfahren sowie das damit herstellbare erfindungsgemäße Proteinpräparat in Verbindung mit den Zeichnungen nochmals näher erläutert. Hierbei zeigen:

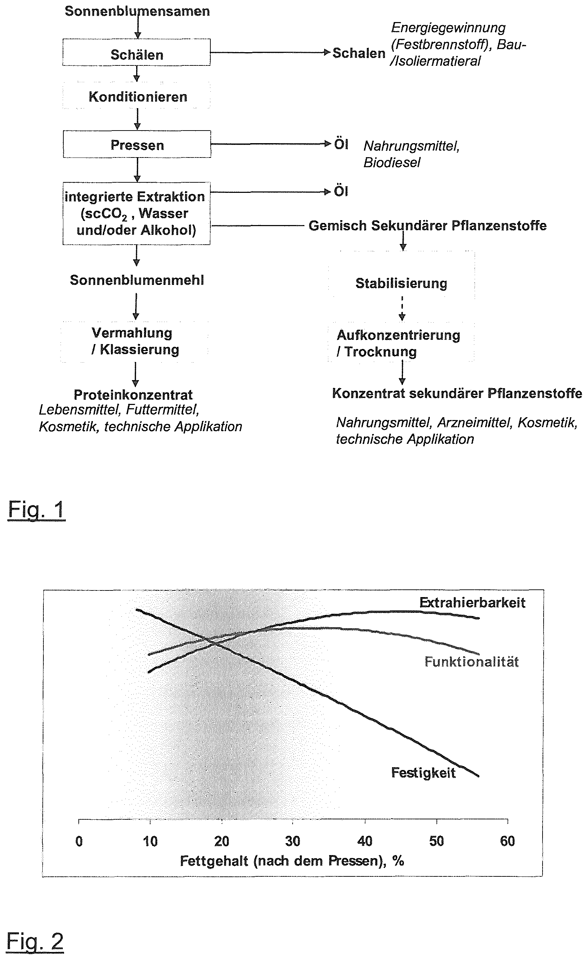

- Fig. 1: schematisch ein Beispiel für den Verfahrensablauf des Verfahrens mit einer Fraktionierung der phenolsäurehaltigen Sonnenblumenkerne in Öl, Polyphenole und Proteinkonzentrat sowie einer optionalen weiteren Fraktionierung in Proteinisolate; die Verwendungsmöglichkeiten der einzelnen Fraktionen sind in kursiver Schrift angegeben;
- Fig. 2: eine Darstellung zur Ermittlung des optimalen Pressgrades hinsichtlich der mechanischen Stabilität und Erhalt der Extrahierbarkeit der Presskuchen und funktionellen Eigenschaften der daraus hergestellten Präparate; und

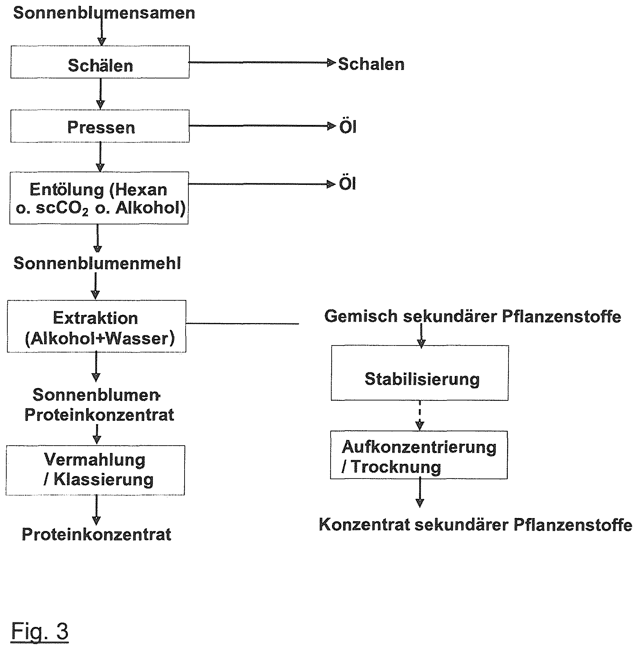

- Fig. 3: schematisch ein weiteres Beispiel für den Verfahrensablauf des Verfahrens mit den Schritten Schälen, Pressen und Hexanextraktion (alternativ: Entölung mit scCO₂ oder mit Alkohol), anschließender Alkohol-Wasser-Extraktion, Alkohol-Wasser-Verdrängungstrocknung und anschließender Feinvermahlung und/oder Sieben.

### Wege zur Ausführung der Erfindung

Das Verfahren zur Herstellung des erfindungsgemäßen Proteinpräparats kann beispielsweise in folgender Weise durchgeführt werden. Als Ausgangsprodukt werden bevorzugt Speisetype-Sonnenblumenkern-Sorten gewählt oder solche, die eine helle Schale haben. Es können aber auch normale und High oleic Öltype-Sonnenblumenkerne verwendet werden.

Der vorbereitete Rohstoff wird in einer Extraktionsvorrichtung nacheinander mit unterschiedlichen Lösungsmitteln unter solchen Bedingungen extrahiert, dass keine oder nur sehr wenig Proteine gelöst werden. Dadurch werden Proteinverluste und -veränderungen minimiert. Besonders vorteilhaft ist die Durchführung einer Lösemittelextraktion mit einem Alkohol, beispielsweise Ethanol, Propanol, Isopropanol.

Selbstverständlich können auch mehrere Extraktionsvorrichtungen für die unterschiedlichen Lösungsmittel eingesetzt werden. Das Gleiche gilt auch für die Trocknungseinrichtungen.

Das gesamte Verfahren umfasst die drei Schritte Auswahl und Aufbereitung der Rohstoffe, mechanische Teilentölung sowie Extraktion, und ist schematisch in Figur 1 dargestellt. Ein weiteres Ausführungsbeispiel ist der Figur 3 zu entnehmen.

### 1. Auswahl und Aufbereitung der Rohstoffe:

Weitgehende Abtrennung der Schalen durch eine geeignete Schältechnologie, so dass der Restschalengehalt in Bezug auf die erhaltene geschälte Kernfraktion bei ≤ 5 Gew%, bevorzugt bei ≤ 1 Gew% liegt. Besonders vorteilhaft werden hierzu gezielt leicht schälbare Rohstofftypen und -sorten ausgewählt, insbesondere Speisekerne anstatt Öltypekerne. Die Angaben zum Restschalengehalt beziehen sich in der vorliegenden Patentanmeldung auf die Gesamtmasse der Kernfraktion, wie sie unmittelbar nach dem Schälen erhalten wird.

Eine gezielte Konditionierung und/oder Trocknung stellt sicher, dass während der Entölung enzymatische Prozesse verhindert oder kontrolliert werden. Diese kann vor oder nach dem Schälen erforderlich sein.

### 2. mechanische Teilentölung

Pressen der schalenarmen Sonnenblumenkerne durch Schneckenpressen, dabei Kontrolle der Temperatur, ggf. Kühlung, auf unter 80°C, besser unter 60 °C, noch besser unter 50°C. Damit werden Maillardreaktionen und sonstige Proteinveränderungen vermindert, außerdem Reaktionen von anderen Begleitstoffen mit Proteinen, z.B. Polyphenole.

Das Pressen erfolgt bis auf einen Restölgehalt von 10 - 35 Gew.%, vorzugsweise 12 - 25% Gew.%, besonders bevorzugt zwischen 17 und 25 Gew.%. Die Pressung wird mit einer Pressgeometrie bzw. Düse durchgeführt, die die Ausformung von stabilen Presskuchen ermöglicht, z.B. in der Form von Pellets oder Strängen, die dennoch nicht zu hart zusammengepresst sind und eine gewisse Porosität aufweisen.

Bei einer geeigneten Wahl der Presskonfiguration (insb. der Düsengeometrie) halten die Pellets bei den oben angegebenen Restölgehalten überraschenderweise sehr gut zusammen und ermöglichen trotz des geringen Schalenanteils ohne anschließende Zerkleinerung eine weitere Entölung mit einem Lösungsmittel. Die Erfinder haben hierbei herausgefunden, dass durch die obige mechanische Teilentölung in Schneckenpressen ein guter Saat-Aufschluss und eine vorteilhafte Produktform für die Extraktion erreicht werden, so dass auf eine weitere Zerkleinerung oder Vorbereitung für die anschließenden weiteren Extraktionsschritte verzichtet werden kann. Dabei wurde erkannt, dass der erzielte Restölgehalt mit den mechanischen Eigenschaften in der Art zusammenhängt, dass es einen optimalen Grad der Entölung gibt, bei dem die Presskucheneigenschaften ideal sind, wie dies aus Figur 2 ersichtlich ist. Der optimale Grad der Entölung liegt in diesem Beispiel bei einem Restölgehalt von etwa 17-20 Gew.%.

Besonders vorteilhaft für die Effizienz der weiteren Extraktionsschritte wird die mechanische Teilentölung bis auf einen Fett- oder Ölgehalt der der geschälten Sonnenblumensamen durchgeführt, bei dem durch das Pressen ein stabiler Presskuchen erhalten wird, der eine Dicke im Bereich zwischen 0,2 und 4 cm, vorzugsweise im Bereich zwischen 0,5 und 2 cm aufweist.

Die Pressung wird mit einer Pressgeometrie bzw. Düse durchgeführt, die die Ausformung von stabilen Presskuchen, z.B. in der Form von Pellets oder Strängen ermöglicht. Besonders vorteilhaft wird eine Schneckenpresse mit einer Rundloch-Matrize oder einer Düse oder ein Extruder mit Runddüse zur Pressung verwendet, so dass die erhaltenen Presslinge als Stränge mit rundem Querschnitt von 5 - 20 mm Durchmesser erhalten werden. Durch die Wahl eines geeigneten Pressgrads, bei dem der Restfettgehalt im Bereich zwischen 12 und 25 % liegt, werden Presslinge mit einer für die Extraktion noch ausreichenden Porosität und guter Festigkeit erhalten. Dabei werden Pressstränge mit einer Bruchfestigkeit zwischen 2 und 10 N/mm², idealerweise zwischen 4 und 8 N/mm² bei einem Schüttgewicht zwischen 300 und 500 kg/m³ erhalten

### 3. Extraktion der vorbereiteten Samen bzw. der Presskuchen-Pellets:

Vorzugsweise erfolgt die weitere Extraktion durch Kombination von mindestens zwei Extraktionslösungsmitteln unterschiedlicher Polarität in der Art, dass enthaltene hydrophilere Begleitstoffe vor, mit oder nach dem Öl extrahiert werden. Als Extraktionslösungsmittel werden nachfolgend alle reinen Fluide und Lösungen (z.B. organische Lösungsmittel oder Wasser und wässrige Lösungen oder überkritische Gase) und Fluidgemische bezeichnet, die zur Extraktion verwendet werden. Dabei werden mindestens zwei Polariätswechsel durch Aufeinanderfolge der Extraktionslösungsmittel eingestellt. Diese können schlagartig oder kontinuierlich eingestellt werden, indem das vorher vorhandene Extraktionslösungsmittel mit dem nachfolgenden vermischt oder durch dieses verdrängt wird. Als solche kommen alle lebensmittelrechtlich zugelassenen Lösungsmittel und ihre Gemische in Frage, insbesondere Wasser, Säuren, Alkohole, Ester, Ketone, z.B. Aceton, Ether, Alkane wie n-Hexan und iso-Hexan, deren Polarität bzw. Wasserlöslichkeit in der genannten Reihenfolge grundsätzlich abnimmt (von hydrophil zu lipophil), sowie überkritische Flüssigkeiten und Gase, z.B. scCO₂ (überkritisches CO₂), das am kritischen Punkt eher lipophil ist und dessen Polarität durch weitere Erhöhung des Drucks in Richtung hydrophil sowie durch Erhöhung der Temperatur weiter verändert werden kann.

So können beispielsweise folgende Schritte durchgeführt werden, wobei die Reihenfolge der hydrophilen und lipophilen Schritte vorzugsweise so gewählt wird, dass die Gesamtextraktion eine maximale Ausbeute ergibt (d.h. mindestens 90% der mit dem reinen Lösungsmittel erzielbaren Ausbeute):
- Extraktion von mäßig hydrophilen Begleitstoffen, insbesondere Phenolsäuren und Aromastoffen, durch Alkohol, vorzugsweise Isopropanol, Ethanol oder Methanol, in einer Konzentration, bei der die Proteine nicht oder nur wenig gelöst werden. Dazu wird eine Alkoholkonzentration von größer 60%, vorzugsweise zwischen 60 und 80%, eingestellt (v/v-Konzentration des Alkohols im Extraktionslösungsmittel). Weiterhin kann auch scCO₂ als Lösungsmittel eingesetzt werden, vorzugsweise bei einer Temperatur zwischen 40 und 80°C und bei einem Druck über 300 * 10⁵ Pa, vorzugsweise im Bereich von 350 - 800 * 10⁵ Pa, wobei es mit zunehmendem Druck hydrophilere Eigenschaften aufweist.
- Extraktion lipophiler Bestandteile mit einem lipophilen Lösungsmittel bis zur vollständigen Entölung auf einen Restölgehalt von höchstens 5% (Büchi-Methode nach Caviezel). Als lipophiles Lösungsmittel kann beispielsweise Hexan, reiner Alkohol (≥ 95%) oder scCO₂ bei einer Temperatur im Bereich von 31 - 60°C und einem Druck im Bereich von 74 - 350 * 10⁵ Pa eingesetzt werden. Damit werden insbesondere Öl, Phospholipide und andere lipophile Bestandteile wie beispielsweise Carotinoide extrahiert.
- ggf. Wiederholung der ersten Extraktion nach der zweiten Extraktion.

Die lipophile Extraktion kann bei Bedarf auch vor der hydrophilen Extraktion durchgeführt werden.

Vorteilhafterweise wird die Polarität der Extraktionslösungsmittel durch vorhandenes Restwasser nach der Vorbehandlung, insbesondere nach der mechanischen Vor-Entölung bzw. während der Ölextraktion verändert, so dass während des Extraktionsprozesses mit einem einzigen zugesetzten Lösungsmittel unterschiedliche Polaritäten des tatsächlichen Extraktionsgemischs entstehen.

Bei Nutzung von überkritischen Gasen, insbesondere überkritischem Kohlenstoffdioxid (scCO₂) kann die Polarität allein durch Änderung von Druck und Temperatur verändert werden, so dass der Zusatz eines weiteren Lösungsmittels für einen Polaritätswechsel nicht erforderlich ist. Durch sukzessive Verdrängung des rohstoffgebundenen Wassers kann die Polarität während der Extraktion nahezu kontinuierlich verändert werden.

Besonders vorteilhaft wird die hydrophilere Polarität zuerst eingestellt, so dass das im Rohstoff gebundene Restwasser zur Modifikation der Polarität in der Art genutzt werden kann, dass hydrophile Substanzen ohne weiteren Wasserzusatz oder mit sehr wenig zugesetztem Wasser extrahiert werden können. Überraschenderweise bewirkt dies beim Übergang zur lipophilen Extraktionsphase gleichzeitig eine Verminderung des Restwassergehalts, so dass die lipophile Extraktion begünstigt wird. Durch die Entfernung des Wassers vor bzw. während der ersten Extraktion kann eine sonst übliche Trocknung vor der Entölung entfallen. Normalerweise wäre nach dem Pressen eine Konditionierung erforderlich, weil bei angestiegenem relativen Wassergehalt im Presskuchen wegen verminderten Ölgehalts und resultierender geringerer Gesamtmasse die Entölung mit lipophilem Lösungsmittel erschwert wäre.

Besonders vorteilhaft wird das erste Lösungsmittel oder werden Reste des ersten Lösungsmittels, z.B. des Alkohols oder Alkohol-Wassergemischs, mit dem anschließenden zweiten Lösungsmittel ausgetrieben.

Ggf. kann es nötig sein, zuvor das Wasser mit Alkohol vollständig zu verdrängen, um nachteilige Einflüsse von Wasser in der nachfolgenden Extraktion zu verhindern. Die Alkoholkonzentration wird in der ersten Extraktion soweit erhöht, dass der Alkohol anschließend durch das lipophilere Lösungsmittel gelöst werden kann. Eine selektive Abtrennung bei unterschiedlichen Druckstufen beim Einsatz von scCO₂ ermöglicht die weitgehende Abtrennung der Alkoholphase. Durch das im Rohstoff enthaltene Wasser oder zugesetztes Wasser oder andere Co-Solvents können die Lösungseigenschaften weiter modifiziert werden, so dass die Gewinnung mäßig polarer Wertstoffe möglich ist. Durch Kombination von hohem Druck (>500*10⁵ Pa) und einer Temperatur zwischen 40 und 60°C werden bessere Extraktionsraten der Phenolsäuren und der Ölbegleitstoffe erzielt. Die Einbringung des zweiten Lösungsmittels (außer Wasser) in die überkritische Phase kann die Extraktion der Phenolsäuren und weiterer Begleitstoffe, z.B. Pigmente und Aromastoffe auch verbessern. Besonders vorteilhaft werden die Extraktionsbedingungen mit scCO₂ so eingestellt, dass hintereinander sowohl Wasser- als auch Alkohol-Reste aus dem Raffinat ausgetrieben werden können und ein Desolventieren mit hohen Temperaturen dadurch überflüssig wird. Dadurch kann auf eine anschließende Trocknung des Raffinats auch bei Verwendung von Wasser als Schleppmittel/ Modifier verzichtet werden.

Bei der Verwendung von wässrigem Alkohol wird die Extraktion mittels des Extraktionslösungsmittels in mehreren Extraktionsschritten durchgeführt, wobei bei mindestens dem letzten Übergang von dem einem zum nächsten Extraktionsschritt der Alkoholgehalt im Extraktionslösungsmittel maximal erhöht wird, d.h. bis zur Konzentration des wässrigen Azeotrophs, z.B. 96 % (v/v) bei Ethanol, so dass die Alkoholkonzentration im Extraktionsgemisch auf über 90 % (v/v) ansteigt. Dies erlaubt es, die anschließende Trocknung besonders schonend zu gestalten, da der Anteil des zu entfernenden Restwassers, das weniger schnell und bei einer höheren Temperatur als der Alkohol verdampft, reduziert ist.

Selbstverständlich kann die weitere Extraktion (nach der mechanischen Teilentölung) auch mit nur einem Lösungsmittel, insbesondere Hexan, durchgeführt werden, um das entfettete proteinhaltige Mehl zu erhalten.

Die Extraktion wird unter solchen Bedingungen durchgeführt, dass Proteine nicht oder nur geringfügig in Lösung gehen und die Proteine nicht oder nur minimal geschädigt werden und keine oder nur wenige unerwünschte chemische Reaktionen auftreten wie Maillardreaktion oder Michael-Addition von Phenolsäuren (z.B. messbar als max. 20% weniger freie Phenolsäuren und/oder verfügbares Lysin und/oder reduzierbare Zucker bzw. max. 10% mehr Lysinoalanin oder Maillardprodukte). Weiterhin treten bei den eingestellten Temperaturen keine thermisch bedingten Aromaveränderungen des extrahierten Materials auf. Hierzu wird insbesondere die Temperatur unter 80°C, besser bei ≤ 60°C gehalten, idealerweise unter 40°C. Wenn eine Hexanentölung durchgeführt wird, kann die vollständige Desolventierung durch Anlegen eines Vakuums (100 - 800 hPa, vorzugsweise 200 - 500 hPa, besonders bevorzugt 200 hPa) verbessert werden, wodurch die Desolventierung bei Temperaturen bis maximal 60°C ermöglicht wird. Bei anderen Lösungsmitteln ist die Anwendung von Vakuum ggf. ebenfalls vorteilhaft, um die Desolventierung bei niedrigeren Temperaturen zu ermöglichen.

Dabei zeigt sich, dass bei Sonnenblumenproteinen eine definierte Denaturierung von 5% - 40%, besonders günstig zwischen 10% und 30% (z.B. messbar als Abweichung um maximal 30%, besser 20%, noch besser 10% hinsichtlich funktioneller Eigenschaften wie Proteinlöslichkeit, max. 30% größere Proteindenaturierung, messbar mit thermoanalytischen Methoden wie DSC), - bezogen auf Proteine des Eingangsprodukts des vorgeschlagenen Verfahrens - besonders vorteilhaft ist, um ein breites Applikationsspektrum zu erhalten.

Die Reihenfolge der Extraktion kann auch umgekehrt erfolgen, wenn z.B. die Extraktstoffe für spezifische Anwendungen nutzbar gemacht werden sollen. Die vollständige Entölung im ersten Schritt kann im Hinblick auf die Gewinnung der Begleitstoffe als funktionelle Lebensmittelzutaten oder für kosmetische oder technische Anwendungen vorteilhaft sein. Besonders vorteilhaft ist die Kombination von Entölung mit überkritischem CO₂, anschließender (wässrig-)alkoholischer Lösungsmittelextraktion und abschließender scCO₂-Behandlung zur gleichzeitigen Desolventierung und Trocknung zu stabilen Endprodukten. Die Kombination wird vorzugsweise so gestaltet, dass alle Extraktionen hintereinander in einem Behälter durchgeführt werden und nur die Lösungsmittel, Temperaturen und Drücke verändert werden.

Überraschenderweise können dabei gleichzeitig die wertvollen Fraktionen Proteine und Phenole gewonnen und beide für unterschiedliche Lebensmittelanwendungen genutzt werden. Die im Alkohol enthaltenen Begleitstoffe können direkt für hochwertige Anwendungen genutzt werden oder weiter verarbeitet werden. Besonders vorteilhaft ist auch die Verwendung von scCO₂ vor oder bei der Extraktion von Polyphenolen, weil durch die Verdrängung von Sauerstoff eine Oxidation verhindert wird.

Überraschenderweise zeigt sich auch, dass beim Einsatz von Alkohol die Abreicherung der Phospholipide gegenüber einer reinen Hexanextraktion verbessert wurde, wodurch die sensorische Qualität des entölten Mehls weiter verbessert wird.

Weiterhin zeigt sich, dass die Proteinfraktion frei von thermisch bedingten Aromastoffen gewonnen werden kann und die Anwendung in Lebensmitteln durch sensorisch neutrale Proteinpräparate verbessert wird. Gleichzeitig bleiben die funktionellen Eigenschaften der Proteine erhalten.

Durch das oben beschriebene Herstellungsverfahren kann ein Sonnenblumenproteinpräparat gewonnen werden, das sich z. B. gegenüber Proteinisolaten, die durch wässrige Fraktionierung und aufwändige Isolierungsverfahren gewonnen werden, durch ein ausgewogenes Nährwertprofil und technofunktionelles Spektrum auszeichnen. Das Proteinpräparat ist auch ohne weitere Aufbereitung, um z. B. den hohen Proteingehalt eines Proteinisolates zu erhalten, u. a. als Lebens- oder Futtermittelzutat geeignet. Überraschenderweise zeigt das Proteinpräparat, obwohl es kein Proteinisolat ist, technofunktionelle Eigenschaften von Proteinisolaten auf. Es hat eine neutrale, helle Farbe und ist weitgehend frei von sensorisch störenden und antinutritiven Begleitstoffen. Insbesondere weist das Sonnenblumenproteinkonzentrat nahezu keinen Eigengeruch und Eigengeschmack auf.

Besonders überraschend ist, dass bereits das entölte Sonnenblumenproteinmehl (SBPM) eine überaus ansprechende Farbe und sehr ausgeprägte Funktionalität hat und für zahlreiche Lebensmittel- und Futtermittelanwendungen geeignet ist.

Nachfolgend wird zur quantitativen Charakterisierung der hergestellten Proteinpräparate auf folgende Bestimmungsverfahren zurückgegriffen:
- Proteingehalt:
   Der Proteingehalt ist definiert als der Gehalt, der sich aus der Bestimmung des Stickstoff und dessen Multiplikation mit dem Faktor 6,25 errechnet. Der Proteingehalt ist z. B. in Prozent bezogen auf die Trockenmasse (TS) angebbar.
- Farbe:
   Die wahrnehmbare Farbe ist mittels CIE-L*a*b*-Farbmessung definiert (vgl. DIN 6417). Dabei gibt die L*-Achse die Helligkeit an, wobei Schwarz den Wert 0 und Weiss den Wert 100 hat, die a*-Achse beschreibt den Grün- oder Rotanteil und die b*-Achse den Blau- oder Gelbanteil.
- Proteinlöslichkeit:
   Die Proteinlöslichkeit ist mittels Bestimmungsverfahren nach Morr et al. 1985 bestimmt (siehe den Zeitschriftenartikel: Morr C.V., German, B., Kinsella, J.E., Regenstein, J.M., Van Buren, J.P., Kilara, A., Lewis, B.A., Mangino, M.E, "A Collaborative Study to Develop a Standardized Food Protein Solubility Procedure. Journal of Food Science", Band 50 (1985) Seiten 1715-1718).Dabei wird das Proteinpräparat zu einem Masse-Volumenanteil von 1:25 bis 1:50 (w/v) (d. h. 1-2 g des Proteinpräparats auf 50 ml Lösung) in einer 0,1 M NaCl-Lösung bei Raumtemperatur suspendiert und unter Verwendung von 0,1 M HCl- oder NaOH-Lösung ca. 60 min bei einem pH-Wert von pH 7 gehalten und mit ca. 200 U/min gerührt und das unlösliche Sediment danach für 15 min bei 20tausendfacher Erdbeschleunigung (20'000g) abzentrifugiert. Die Proteinlöslichkeit ist z. B. in Prozent angebbar, wobei eine Proteinlöslichkeit von x % bedeutet, dass x % des im Präparat vorhandenen Proteins im geklärten Überstand wiedergefunden werden, wenn die genannte Methode angewendet wird.
- Wasserbindung:
   Das Wasserbindevermögen ist mittels Bestimmungsverfahren (nachfolgend AACC-Bestimmungsverfahren genannt) definiert, wie es angegeben ist in: American Association of Cereal Chemists, "Approved methods of the AACC". 10th ed., AACC. St. Paul, MN, 2000b; Method 56-20. "Hydration capacity of pregelatinized cereal products". Das Wasserbindevermögen ist z. B. in ml/g angebbar, d. h. Milliliter gebundenes Wasser pro Gramm Präparat, und wird gemäss dem AACC-Bestimmungsverfahren bestimmt über das Gewicht des mit Wasser gesättigten Sediments abzüglich der Einwaage des trockenen Präparats nach Mischung von ca. 2 g Proteinpräparat mit ca. 40 ml Wasser für 10 Minuten und Zentrifugation bei 1'000g für 15 Minuten bei 20°C.
- Ölbindung:
   Das Ölbindevermögen ist mittels Bestimmungsverfahren (nachfolgend Fettbinde-Bestimmungsverfahren genannt) definiert, wie es angegeben ist in: Ludwig I., Ludwig, E., Pingel B., "Eine Mikromethode zur Bestimmung der Fettbindkapazität". Nahrung/Food, 1989, 33(1), 99.
   Das Ölbindevermögen ist z. B. in ml/g angebbar, d. h. Milliliter gebundenes Öl pro Gramm Präparat, und wird gemäß o.g. Bestimmungsverfahren gemessen als Volumen des ölbindenden Sediments nach Mischung von 1,5 g Proteinpräparat mit 15 ml Maiskeimöl für 1 Minute und Zentrifugation bei 700g für 15 Minuten bei 20°C.
- Emulgierkapazität:
   Die Emulgierkapazität ist mittels Bestimmungsverfahren (nachfolgend Konduktivitätsmessungs-Methode genannt) bestimmt, bei welchem einer 1 %igen Suspension des Proteinpräparats von 100 ml, pH 7, Maiskeimöl zugegeben wird bis zur Phaseninversion der Öl-in-Wasser-Emulsion. Die Emulgierkapazität ist definiert als das maximale Ölaufnahmevermögen dieser Suspension, bestimmt über die spontane Abnahme der Leitfähigkeit bei der Phaseninversion (vgl. den Zeitschriftenartikel von Wäsche, A., Müller, K., Knauf, U., "New processing of lupin protein isolates and functional properties". Nahrung/Food, 2001, 45, 393-395) und ist z. B. angebbar in ml Öl/g, d. h. Milliliter emulgiertes Öl pro Gramm Proteinpräparat
- Schaumaktivität:
   Die Schaumaktivität ist angegeben in Prozent, gemessen als Volumenzunahme einer 5 %igen Lösung, pH 7, bei Aufschlag während 8 min auf Stufe 3 (591 U/min) in einer Hobart 50N Standard-Küchenmaschine (Stahlkessel mit 5 Liter Inhalt) mit Rührbesen (Drahtbesen).
- Schaumdichte:
   Die Schaumdichte ist angegeben in g/l, d. h. Masse des Schaums pro Volumeneinheit, und wird gemessen nach Aufschlag einer 5 %igen Lösung, pH 7, von 8 min auf Stufe 3 (591 U/min) in einer in einer Hobart 50N Standard-Küchenmaschine (Stahlkessel mit 5 Liter Inhalt) mit Rührbesen (Drahtbesen).
- Schaumstabilität:
   Die Schaumstabilität ist angegeben in Prozent, gemessen als Volumenabnahme von 100 ml Schaum innerhalb einer Stunde nach Aufschlag einer 5 %igen Lösung, pH 7, 8 min auf Stufe 3 (591 U/min) in einer Hobart 50N Standard-Küchenmaschine (Stahlkessel mit 5 Liter Inhalt) mit Rührbesen (Drahtbesen).
- Fettgehalt:
   Der Fettgehalt ist bestimmt nach Probenaufschluss und Verseifung der Fettsäuren z. B. nach der Caviezel-Methode (beschrieben bei DGF. "Method of Caviezel", DGF K-I 2c (00). In Deutsche Gesellschaft für Fettwissenschaft e. V., Münster. DGF-Einheitsmethoden, 2nd edition. Stuttgart: WVG, 2004).
- Für Vergleichszwecke wurden folgende kommerzielle hergestellte Produkte verwendet:
   - Erbsenproteinisolat Pisane® (hergestellt von Cosucra),
   - Sojaproteinisolat SUPRO® EX33 (hergestellt von DuPont).
   - Natriumkaseinat (sprühgetrocknet), FN5S von Rovita.

Mit dem Herstellungsverfahren sind erfindungsgemäße Proteinpräparate aus Sonnenblumenkernen mit folgenden Eigenschaften herstellbar:
Aussehen:
   - In schüttfähiger Form, z. B. als Flocken, Granulat, Pulver oder in Form anderer Partikel.
   - Die Farbe ist weiß bis cremefarben, hellgrau oder hellgelb, gegebenenfalls mit einem Anteil heller oder dunkler gefärbter Partikel von maximal 5 % w/w, vorzugsweise unter 2 % w/w. Die Helligkeit L* gemäß CIE-L*a*b*-Farbmessung ergibt einen Wert von mindestens 80, L* >= 80. Folgendes sind typische Werte für L*, a* und b*:
      L* >= 80, -5 < a* < +5, -5 < b* < +20; vorzugsweise
      L* >= 85, -3 < a* < +3, - 2 < b* < +15; besonders bevorzugt L* >= 90, -1 < a* < +1, 0 < b* < +10.
Zusammensetzung:
   - Der Proteingehalt beträgt weniger als 90 % in der Trockenmasse (TS), vorzugsweise weniger als 80 % bezogen auf TS. Typischerweise liegt der Proteingehalt zwischen 50 und 70 % bezogen auf TS.
   - Gesamtballaststoffgehalt zwischen 10 und 40 % bezogen auf TS, bevorzugt zwischen 10 und 30 % bezogen auf TS.
   - Fettgehalt, ermittelt z B. durch gravimetrische Bestimmung nach Soxhletextraktion, unter 3 % bezogen auf TS, bevorzugt unter 1 %.
   - Gesamtzuckergehalt unter 15 % bezogen auf TS, bevorzugt unter 5 %, besonders bevorzugt unter 2 %.
   - Gehalt an unerwünschten, insbesondere antinutritiven Stoffen:
      - Phytinsäuregehalt unter 5 % bezogen auf TS, bevorzugt unter 2 %, besonders bevorzugt unter 1 % .
      - Raffinosegehalt unter 5% bezogen auf TS, bevorzugt unter 2,5%, besonders bevorzugt unter 0,5%.
      - Phenolsäuregehalt (bestimmt als Chlorogensäure) unter 5 % bezogen auf TS, bevorzugt unter 2 %, besonders bevorzugt unter 0,5 %.
   - Ligningehalt unter 6 % bezogen auf TS, bevorzugt unter 4 %, besonders bevorzugt unter 3 %.
   - Allgemein ist der Protein- sowie Ligningehalt beim Sonnenblumenproteinmehl (SBPM) tiefer als beim daraus hergestellten Sonnenblumenproteinkonzentrat (SBPK), während der Gehalt an Fett, Zuckern und Phenolsäuren beim SBPM höher als beim SBPK ist.
Technofunktionelle Eigenschaften:
   - Proteinlöslichkeit:
      Die Proteinlöslichkeit, bestimmt nach dem PNG-Bestimmungsverfahren, ist größer als 30 %, bevorzugt größer als 40 %. Sie liegt im Bereich von 30-60 %.
   - Wasserbindung:
      Die Wasserbindung, bestimmt nach dem AACC-Bestimmungsverfahren, beträgt mindestens 2 ml/g, bevorzugt mindestens 3 ml/g. Vergleichsmessungen zeigen, dass die Wasserbindung des Präparats mindestens 30 % der Wasserbindung von Pisane®, bestimmt nach dem AACC-Bestimmungsverfahren, beträgt.
   - Ölbindung:
      Die Ölbindung, bestimmt nach dem Fettbinde-Bestimmungsverfahren, beträgt mindestens 1 ml/g, bevorzugt mindestens 4 ml/g. Vergleichsmessungen zeigen, dass die Ölbindung mindestens 100% der Ölbindung von Pisane® oder von Supro® EX33, bestimmt nach demselben Verfahren, beträgt.
   - Emulgierkapazität:
      Die Emulgierkapazität, bestimmt nach der Konduktivitätsmessungs-Methode, beträgt mindestens 400 ml Öl/g, bevorzugt mindestens 500 ml Öl/g. Vergleichsmessungen zeigen, dass die Emulgierkapazität mindestens 40% der Emulgierkapazität von Natriumkaseinat FN5S, bestimmt nach demselben Verfahren, beträgt.
   - Schaumbildende Eigenschaften:
      - Schaumaktivität:
         Die Schaumaktivität beträgt mindestens 1000 %. Vergleichsmessungen mit frischem Hühnereiklar bei Aufschlag während 3 min auf Stufe 3 in einer Hobart 50N Standard-Küchenmaschine mit Rührbesen zeigen, dass die Schaumaktivität des Proteinpräparats mindestens 50 % oder sogar mindestens 60 % der Schaumaktivität von Hühnereiklar entspricht.
      - Schaumdichte:
         Die Schaumdichte liegt im Bereich von 80 und 110 g/l. Vergleichsmessungen mit Hühnereischnee nach Aufschlag während 3 min auf Stufe 3 in einer Hobart 50N Standard-Küchenmaschine mit Rührbesen zeigen, dass die Schaumdichte im Bereich von 80 und 110 % der Schaumdichte von Hühnereischnee liegt.
      - Schaumstabilität:
         Die Schaumstabilität beträgt mindestens 80 %, vorzugsweise mindestens 90 %. Sie entspricht typischerweise mindestens 90 % der Schaumstabilität von Hühnereischnee, gemessen als Volumenabnahme von 100 ml Hühnereischnee innerhalb einer Stunde nach Aufschlag während 3 min auf Stufe 3 in einer Hobart 50N Standard-Küchenmaschine mit Rührbesen.
Sensorische Eigenschaften:
   Nebst der hellen Farbe ist das Proteinpräparat, insbesondere in Form des SBPK, im Wesentlichen geruchsfrei und geschmacksneutral. Insbesondere fehlen im Wesentlichen die pflanzen- oder saateigenen Aromen. So sind im Wesentlichen kein bohniger und grasiger Geruch und Geschmack sowie im Wesentlichen kein Bittergeschmack wahrnehmbar.

Sensorische Tests, in welchen geschulte Prüfer einen bestimmten Geschmacks- oder Aromaeindruck des Proteinpräparats und einer geeigneten Referenzsubstanz vergleichen und auf einer Skala von 1 bis 10 (1 = nicht wahrnehmbar, 10 = stark wahrnehmbar) bewerten, wobei die Referenzsubstanz so gewählt ist, dass bei ihr der zu prüfende Geschmacks- oder Aromaeindruck mit mindestens 8 bewertet wird, zeigen, dass dem Proteinpräparat ein Wert von 3 oder weniger (typischerweise ein Wert von 1) zugeordnet wird.

Beispiele von zu testenden Geschmacks- oder Aromaeindrücken sind:
- bohniger Geschmack im Vergleich zu Sojabohnen;
- Grüner bis grasiger Geschmack im Vergleich zu grünem Paprika oder grünen Erbsen;
- Bittergeschmack im Vergleich zu einer 0,1 %igen wässrigen Koffeinlösung.

Farbe, Eigengeschmack und Eigengeruch des Proteinpräparats sind so, dass bei der Einarbeitung in Lebens- und Futtermittel im Wesentlichen keine mit üblichen statistischen Methoden ermittelte signifikante, als negativ zu wertende Veränderung des arteigenen Aussehens, Geruchs und Geschmacks der fertigen Zubereitung auftritt.

Sensorische Tests zeigen, dass die Geschmacks- und Aromaänderung, die in einer Lebensmittelzubereitung durch den Einsatz des Proteinpräparats hervorgerufen wird, gegenüber der Lebensmittelzubereitung ohne das Proteinpräparat auf ein derartiges Maß begrenzt ist, dass ein geschulter Prüfer eine Abweichung eines der oben genannten Geschmacks- oder Aromamerkmale auf einer Skala von 1-10 von maximal 3 Stufen, besser maximal 1 Stufe (fast nicht mehr erkennbare Abweichung) erkennen kann.

Bei dem Verfahren wird durch die Anwendung von mit dem saateigenen Wasser vermischtem Alkohol oder wässrigen Alkohollösungen der Großteil der pflanzeneigenen Aromastoffe und weiteren sekundären Pflanzenstoffe wie Phenolsäuren entfernt. Dadurch werden helle, verfärbungsstabile und nahezu geruchs- und geschmacksneutrale Mehle erhalten.

Überraschenderweise kann der Proteingehalt des Raffinats/ Raffinats/Mehls auf diese Weise durch die Mitextraktion anderer niedermolekularer Bestandteile, insbesondere die enthaltenen Zucker, auf Anteile größer/gleich 60% erhöht werden, so dass ohne weitere Prozessschritte hochwertige stabile Proteinkonzentrate erhalten werden.

In der Alkohol-, wässrigen oder Wasser-AlkoholPhase fällt dabei ggf. ein Gemisch von Zuckern/ Oligosacchariden und sekundären Pflanzenstoffe wie Phenolsäuren an. Besonders vorteilhaft können die Zuckerstoffe als Trägerstoff für die phenolischen Stoffe z.B. bei einer anschließenden Trocknung zur Herstellung einer Anwendungsform dienen. Durch selektive Adsorption, Kristallisation oder Fällung können die beiden Fraktionen weiter gereinigt oder aufgetrennt werden, um beide Fraktionen getrennt nutzbar zu machen.

Die Erfinder haben außerdem erkannt, dass sich bei einer Extraktion mit scCO₂ besondere Vorteile ergeben bei der weiteren nasstechnischen Verarbeitung der entölten Schrote bzw. Mehle durch den verminderten Gehalt an störenden Begleitstoffen, weil das im Schrot enthaltene CO₂ gleichzeitig in einer nachfolgenden Verarbeitung stabilisierend wirkt, da Oxidationsprozesse eingeschränkt werden.

Ein weiterer Vorteil ergibt sich, wenn die gewonnenen Präparate nach der scCO₂-Behandlung direkt abgepackt werden. Überraschenderweise sind sie dann ohne weitere Schutzgaszugabe direkt vor Oxidation geschützt. Eine Teilbelüftung oder Kombination mit anderen Schutzgasen kann dennoch vorteilhaft sein.

Weiterhin wurde erkannt, dass die funktionellen Eigenschaften der Proteinpräparate durch die Einstellung der Korngröße modifiziert werden können. Durch eine entsprechende Feinzerkleinerung oder Fraktionierung nach Korngröße oder Korndichte des Sonnenblumenproteinmehls oder des Konzentrats können die Wasserbindung sowie die Emulgierkapazität gezielt eingestellt werden, um unterschiedliche Anforderungen zu erfüllen. Besonders vorteilhaft wird dabei eine Korngröße von ≤ 500 µm eingestellt oder eine Fraktion mit einer Korngröße ≤ 500 µm abgetrennt.

Mit dem Verfahren ist mit einem minimalen Einsatz von Wasser die Herstellung hochwertiger Sonnenblumenproteinpräparate möglich, die überraschenderweise ähnlich gute Eigenschaften wie Proteinisolate aufweisen, obwohl sie einen geringeren Proteingehalt haben. Mit Hilfe der beschriebenen Technologie werden Sonnenblumenkerne nahezu vollständig in ernährungsphysiologisch und technofunktionell wertvolle Lebensmittelinhaltsstoffe und weitere Fraktionen für eine energetische und technische Nutzung fraktioniert, wobei die Proteinausbeute besonders hoch ist.

So wurde auch erkannt, dass die anfallende alkoholische zuckerhaltige Lösung direkt für die Fermentation zu Bio-Ethanol verwendet werden kann.

Die Erfinder haben außerdem erkannt, dass das Öl, das durch die unpolare Extraktion gewonnen wird und Reste von Hexan enthält, ohne weitere Aufbereitung für die Beimischung zu oder Herstellung von Biodiesel geeignet ist oder direkt als Brennstoff genutzt werden kann.

In einer anderen vorteilhaften Ausgestaltung des Verfahrens werden die Extraktionen so hintereinander geschaltet, dass mit dem überkritischen CO₂ gleichzeitig der im Schrot gebundene Restalkohol extrahiert wird, so dass nachfolgende Destillations- oder Reinigungsschritte entfallen. Die Erfinder haben festgestellt, dass das Öl, das durch die unpolare Extraktion gewonnen wird und Reste von Alkohol enthält, sich erstaunlicherweise sehr gut zur Weiterverarbeitung zu Biodiesel eignet und in einem Prozess, der auf enzymatischer Umesterung von Fett mit Alkohol beruht, direkt eingesetzt werden kann. Daher werden besonders vorteilhaft eventuell in das Öl verschleppte Alkoholanteile nicht entfernt sondern bleiben enthalten und werden bei einer weiteren Verarbeitung des Öls zu Biodiesel nach dem Umesterungsverfahren genutzt.

### Ausführungsbeispiele

### Beispiel 1: Sonnenblumenkernproteinkonzentrat aus alkoholischer Extraktion aus entölten, geschälten Sonnenblumenkernen

Geschälte Speisekerne mit einer Reinheit von 99,9%, d.h. einem Schalenanteil von < 0,1%, wurden in einer Schneckenpress mit einer Düse von 5mm Durchmesser bei einer Temperatur von ca. 40°C (+-5°C) auf einen Restfettgehalt von 23 % entölt und die erhaltenen strangförmigen Presslinge in einem Soxhlet für 36h mit n-Hexan entölt und bei Raumtemperatur luftgetrocknet, um Hexanreste zu entfernen. Das so erhaltene Hexanentölte phenolsäurehaltige Sonnenblumenschrot aus geschälten Speisekernen wurde im Soxhlet mit Methanol (95%) extrahiert, wobei die Alkoholkonzentration sich durch Extraktion des Wassers von anfänglich ca. 80% (v/v) auf ca. 95% erhöhte. Die Temperatur des Ölbads betrug ca. 85 °C, die Extraktionstemperatur betrug zwischen 20 °C (Kühler) und maximal 65 °C (Siedetemp. Methanol = 65 °C). Nach 12 Durchläufen wurde die Extraktion beendet, nachdem schon mehrere Durchläufe keine gelbliche Färbung des Extrakts mehr erkennbar war.

Das so erhaltene Sonnenblumenproteinkonzentrat ist ein feines helles Pulver mit einem Proteingehalt von > 60 %. Die Zusammensetzung des Sonnenblumenkernmehls als eine Ausgestaltung des erfindungsgemäßen Proteinpräparats sowie des daraus erhaltenen Sonnenblumenproteinkonzentrats und -isolats, die nicht Teil der vorliegenden Erfindung sind, ist in nachfolgender Tabelle angegeben.

| **Nr.** | **Probenbezeichnung** | **Trockenmasse (TS)** | **Protein in TS** | **Fett in TS (Büchi)** | **Asche in TS** | **Phenolsäuren in TS** | **Emulgierkapazität** |
|---|---|---|---|---|---|---|---|
| | | % | % | % | % | % | ml/g |
| 1 | Sonnenblumenkernmehl (Schrot aus geschälten Sonnenblumenkernen) | 90,4 | 61,1 | 3,6 | 7,5 | 0,50 | 510 |
| 2 | Sonnenblumenprotein-konzentrat (aus 1) | 87,8 | 76,5 | 1,5 | 8,6 | 0,01 | 210 |
| 3 | Sonnenblumenproteinisolat (aus 2) | 89,1 | 96,2 | 0,29 | 4,20 | 0,00 | 675 |

Das Proteinkonzentrat ist arm an pflanzeneigenen Aromakomponenten. Die Farbe dieses schalenarmen Sonnenblumenproteinmehls und -konzentrats ist besonders ansprechend bzw. neutral und wird nach CIE L*a*b* mit folgenden Werten wiedergegeben:

| **Nr.** | | **L*** | **a*** | **b*** |
|---|---|---|---|---|
| 1 | Sonnenblumenkernmehl | 90,0 | 0,47 | 6,33 |
| 2 | Sonnenblumenproteinkonzentrat (aus1) | 86,8 | 0,18 | 8,11 |
| 3 | Sonnenblumenproteinisolat (aus 2) | 73,9 | 1,22 | 10,94 |

Das Sonnenblumenmehl enthielt vorher ca. 0,5 % Kaffeesäurederivate, nachgewiesen mit HPLC (elektrochemische Detektion) und quantifiziert mit photometrischer Bestimmung. Das extrahierte Sonnenblumenmehl, nachfolgend Sonnenblumenproteinkonzentrat genannt, enthielt nur noch Spuren von Chlorogensäure, d.h. an der Nachweisgrenze von 0,01%. Es wurden demnach 90% der Phenolsäuren extrahiert, identifiziert und quantifiziert als Kaffeesäurederivate. Die extrahierte Menge wurde vollständig im Extrakt wiedergefunden. Der Trockenmasseverlust (TS) betrug 24%. Die extrahierte Trockenmasse bestand zu geringen Anteilen aus Protein, Fett und Mineralstoffen. Neben Phenolsäuren waren hauptsächlich Zucker, Oligosaccharide, Ballaststoffe enthalten, die insgesamt zu 63% extrahiert wurden, wovon Oligosaccharide wie Raffinose bei vollständiger Extraktion maximal 30% ausmachen. Überraschenderweise gehen also andere sekundäre Pflanzenstoffe, insbesondere Phytinsäure in den Extrakt über.

Die Phenolsäuren wurden fast vollständig aus dem Schrot extrahiert und konnten im Extrakt nachgewiesen werden. Der Mineralstoffgehalt des Schrotes nahm durch die Behandlung mit Methanol geringfügig zu, während übrige Begleitstoffe entfernt wurden. Die Extraktion mit Methanol führt zu einer weitgehenden Abreicherung störender Begleitstoffe, insbesondere der Phenolsäuren sowie von Ölbegleitstoffen. Der Proteingehalt wurde auf über 60% erhöht, so dass ein farbstabiles Proteinkonzentrat gewonnen werden kann oder eine folgende nasstechnische Gewinnung hochwertiger Proteinisolate nicht durch Polyphenole gestört wird (siehe Farbe des Proteinisolats, Tabelle).

### Beispiel 2: Pressen von geschälten Sonnenblumenkernen

Geschälte Speisekerne wurden mit einer Schneckenpresse bei 40 - 50°C mit 3 unterschiedlichen Düsen entölt, die jeweils einen Durchmesser von 6, 5 und 4 mm haben. Die erhaltenen Presskuchen unterschieden sich im Fettgehalt sowie in ihrer Struktur und Farbe (Tabelle 2-1). Der Fettgehalt wurde nach zwei Methoden bestimmt, wobei die Büchi-Methode (nach Caviezel) den Gesamt-Fettgehalt angibt und die Soxtherm-Methode den extrahierbaren Anteil bestimmt.

**Tabelle 2-1**

| **Probenbezeichnung** | **Trockenmasse (TS)** | **Protein in TS** | **Fett in TS (Büchi)** | **Fett in TS (Soxtherm)** |
|---|---|---|---|---|
| | % | % | % | % |
| geschälte Sonnenblumenkerne | 94,7 | 25,2 | 55,9 | 52,2 |
| Presskuchen 1 Düse 6mm | 92,4 | 37,9 | 36,3 | 34,5 |
| Presskuchen 2 Düse 5mm | 92,3 | 37,9 | 35,3 | 33,4 |
| Presskuchen 3 Düse 4mm | 90,3 | 52,8 | 9,6 | 6,6 |

Beim Pressen mit der dünnsten Düse erhöhte sich der Druck in der Presse deutlich, so dass ein sehr fester Presskuchen mit geringem Fettgehalt von ca. 10% erreicht wurde. Der Presskuchen war unter diesen Bedingungen allerdings dunkler, was auf Oxidation oder Maillardreaktion schließen lässt. Bei der schonenderen Pressung mit einer größeren Düse 5/6 mm war der Restfettgehalt mit ca. 33 bzw. 35% wesentlich höher. Allerdings konnte dieser durch nachfolgende Extraktion auf unter 1 % abgesenkt werden (Tabelle 2-1).

Die Bestimmung der funktionellen Eigenschaften ergab eine Verbesserung der Proteinlöslichkeit sowie der Emulgierkapazität gegenüber den Ausgangskernen bei den letzten beiden Presskuchen, d.h. dass ein verbesserter Zellaufschluss und eine gute Porosität durch das Pressen erreicht wurden. Die Porosität geht mit zunehmendem Pressgrad verloren. Um eine optimale Festigkeit des Presskuchens mit einer guten mechanischen Stabilität während der nachfolgenden Extraktion zu erhalten, sollte der Pressgrad dagegen möglichst hoch sein.

Neben der Düsengeometrie wirkte sich auch die Temperatur der Pressung auf den Entölungsgrad und die Struktur der Presslinge aus. Die Festigkeit von Presslingen mit rundem Querschnitt wurde mittels Texture Analyzer (TA) bei einer radialen Druckbeanspruchung mit einem Stempel mit 75 mm Durchmesser bei einer Stempelgeschwindigkeit von 1mm/s bestimmt. Gemessen wurde die maximale Kraft, die aufgewendet wurde bis zum Bruch des Presslings. Die Kraft wurde bezogen auf die beanspruchte Fläche von 1mm Breite und der Länge des Presslings unter dem Stempel Der Mittelwert des Bruchdrucks wurde anhand von jeweils 20 Proben bestimmt.

**Tabelle 2-2**

| | Fettgehalt | Mittelwert Bruchdruck [N/ mm2] | Schüttdichte [kg/m³] |
|---|---|---|---|
| Düse 5mm, gepresst bei < 40°C | 35% | 0,58 | 310 |
| Düse 5mm gepresst bei < 40°C | 23% | 0,96 | 350 |
| Düse 5mm, gepresst bei 55°-60°C | 17% | 4,81 | 420 |
| Düse 8mm, gepresst bei 60-70°C | 11% | 9,76 | 470 |

Ein sehr niedriger Restfettgehalt von 11% konnte auch mit einer größeren Düse mit 8 mm Durchmesser (Tabelle 2-2) erzielt werden, wenn die Temperatur auf bis zu 70°C erhöht wurde. Diese Presslinge wiesen eine sehr hohe mechanische Stabilität auf, hatten jedoch eine hohe Schüttdichte und daher niedrigere Porosität und wiesen eine etwas dunklere Farbe auf als die Presskuchen, die bei 40°C erhalten wurden. Bei Anwendung von niedrigeren Temperaturen unter 60°C konnte ein annähernd festes Material mit jedoch deutlich höherer Porosität erhalten werden (Tabelle 2-2), dass nur wenig thermische Schädigung aufwies und sich sehr gut extrahieren ließ. Dagegen wurden bei Temperaturen > 70°C bereits deutliche Proteinschäden und eine starke Verfärbung festgestellt.

Es wurde erkannt, dass bei einem Pressgrad auf einen Restfettgehalt im Bereich von ca. 15 % bis 25 % Restfett, Presskuchen-Pellets erhalten werden, die trotz fehlender Schalen eine gute mechanische Stabilität bei noch ausreichend vorhandener Porosität aufweisen, so dass ohne weitere Strukturierung oder Zerkleinerung bei der nachfolgenden Extraktion eine vollständige Entölung möglich ist. Überraschenderweise weisen die Pellets auch nach der Entölung trotz der mit der Entfernung des Öls einhergehenden Lockerung der Struktur noch eine ausreichende mechanische Stabilität auf, so dass sie einer Extraktion mit einem weiteren Lösemittel unterworfen und so an proteinfremden Substanzen abgereichert werden können, ohne auseinander zu fallen. Durch die poröse Struktur haben sie ein sehr günstiges Extraktionsverhalten für eine weitere Extraktion, z.B. mit alkoholischer Lösung. Dadurch kann auf eine Strukturierung oder Zerkleinerung vor der Extraktion, die sonst üblicherweise durchgeführt werden muss, verzichtet werden.

Je nach Konfiguration und Geometrie der Pressvorrichtung ist der optimale Pressgrad bei einem Restölgehalt von ca. 15% bis 25 % erreicht (vgl. Fig. 2). Dabei wurde erkannt, dass auch bei Temperaturen unter 60°C eine ausreichende Verdichtung der Presslinge erzielt wird und gleichzeitig die Funktionalität und Farbe der Proteine am besten erhalten bleiben.

Insgesamt können durch ein Pressen, bei welchem ein bestimmter Restfettgehalt bestehen bleibt, die Partikel so strukturiert werden, dass eine nachträgliche Strukturierung oder Zerkleinerung nicht mehr erforderlich ist, die sonst üblicherweise zum Aufbrechen des Presskuchens vor der Extraktion durchgeführt wird. Neben der Vereinfachung des Verfahrens trägt dies zur Schonung des Presskuchens bei, so dass u. a. Proteinfunktionalität und die Farbe im Endprodukt verbesserbar sind.

Auch werden durch den reduzierten Pressgrad die Proteine geschont und die funktionellen Eigenschaften des Proteinpräparats bleiben in verbessertem Masse erhalten. Dabei wird gleichzeitig eine Partikelform hergestellt, die eine optimale Extraktion ermöglicht und so der Restölgehalt nach dem Entölen weiter gesenkt werden kann. Auch dies trägt u. a. zu einer Verbesserung der Farbe des Proteinpräparats bei.

### Beispiel 3: Sonnenblumenkernmehle und Proteinkonzentrate, erhalten durch Entölung und Extraktion von schalenfreien Presskuchen mit Hexan, scCO2 und Ethanol

Herstellung:
1. Schälung der Sonnenblumensamen und Auftrennung in eine Kern- und eine Schalenfraktion und Verwendung der Kernfraktion mit einem Schalengehalt von maximal 0,5% (w/w)
2. mechanische Teilentölung auf einen Restfettgehalt von ca. 36% durch Pressung, wie bei Beispiel 2
3. Entölung des Sonnenblumenpresskuchens a) mit iso-Hexan in einem Perkolator bei Temperaturen von maximal 60°C oder b) Extraktion mit überkritischem CO₂ in einem Druckbehälter (Einstellungen s.u., Tabelle)
4. Extraktion des Presskuchens aus 2 oder des Proteinmehls aus 3a mit Ethanol und/oder Hexan in einer Soxhlet-Apparatur (Einstellungen s.u., Tabelle)
5. Austreiben des Hexans nach der Extraktion 3a mit überhitztem Hexandampf unter Vakuum (< 500 hPa)
6. Austreiben weiteren Hexans aus 5 mit überhitztem Wasserdampf unter Vakuum (< 500 hPa).
7. Entfernung von Lösemittelresten aus 6 durch Erhitzung auf 60 °C im Vakuum (< 500 hPa). Das so erhaltene Raffinat wird nachfolgend Proteinmehl genannt.
8. Entfernung des Lösemittels nach Extraktionen 4 im Luftstrom bei Raumtemperatur, um Proteinkonzentrate zu erhalten.
9. Verdampfung des Alkohols und Trocknung des im Verfahrensschritt 8 erhaltenen Raffinats im Rotationsverdampfer unter Vakuum bei maximal 50°C, um ein Sonnenblumenproteinkonzentrat zu erhalten.
10. Vermahlung der Sonnenblumenproteinmehle und konzentrate aus Schritt 3b, 7 oder 9 in einer Stiftmühle mit Siebeinsatz 0,5 mm, um die Sonnenblumenproteinpräparate als feines Pulver zu erhalten.
11. - Einsatz der Proteinmehle und Proteinkonzentrate mit oder ohne vorherige oder nachfolgende Zerkleinerung.

Die Pellets aus der Schneckenpresse (5-mm-Düse aus Beispiel 2) wurden anschließend auf zwei unterschiedliche Arten entölt, 1. mit Hexan (Entölung und Desolventierung bei Temperaturen unterhalb 60°C) und 2. mit überkritischem CO2. Dabei wurde mit Hexan eine vollständige Entölung erzielt, die Extraktion mit CO2 bei 800*105 Pa war ebenfalls nahezu vollständig, bei 285*105 Pa wurde dagegen knapp 20 % weniger Öl extrahiert (50°C, 100 kg/kg CO2). Die Untersuchung der Säurezahlen der Öle aus beiden Extraktionsverfahren ergab keine grundlegenden Unterschiede. Hiermit wurde außerdem gezeigt, dass sich die Pellets ohne weitere Zerkleinerung oder Aufbereitung sehr gut zur Extraktion eignen.

**Tabelle 3-1**

| **Nr.** | **Probenbezeichnung und Herstellung** | **Trockenmasse (TS)** | **Protein in TS (Nx6,25)** | **Fett in TS (Büchi)** | **Asche in TS** | **EC** |
|---|---|---|---|---|---|---|
| | | % | % | % | % | |
| 1 | Presskuchen | 92,3 | 42,3 | 35,3 | 4,6 | 505 |
| 2 | Sonnenblumenkernmehl Hexan-entölt <60°C (aus 1) | 90,8 | 63,6 | 3,0 | 7,7 | 510 |
| 3 | Sonnenblumenkernmehl scCO2-extrahiert 50°C, 285*10⁵ Pa (aus 1) | 94,2 | 58,6 | 10,7 | 6,7 | 598 |
| 4 | Sonnenblumenkernmehl scCO2-extrahiert 50°C,800*10⁵ Pa (aus 1) | 93,6 | 62,7 | 3,8 | 7,7 | 513 |
| 5 | Sonnenblumenkernproteinkonzentrat mit Ethanol extrahiert (aus 1) | 92,4 | 58,9 | 15,7 | 6,9 | 280 |
| 6 | Sonnenblumenkernproteinkonzentrat mit Ethanol extrahiert (aus 2) | 90,9 | 69,0 | 0,4 | 8,4 | 380 |
| 7 | Sonnenblumenkernproteinkonzentrat mit Ethanol extrahiert und mit Hexan entölt, fließender Übergang (aus 5) | 90,4 | 70,3 | 0,3 | 8,4 | 285 |
| 8 | Sonnenblumenkernproteinkonzentrat mit Ethanol extrahiert, getrocknet und anschließend mit Hexan entölt (aus 5) | 90,3 | 69,0 | 0,2 | 8,2 | 285 |

Eigenschaften:
Die so erhaltenen Sonnenblumenproteinmehle und - konzentrate, von denen die Sonnenblumenmehle Nr. 2 und Nr. 4 Ausgestaltungen des erfindungsgemäßen Proteinpräparats darstellen und die anderen Mehle und Konzentrate nicht Teil der vorliegenden Erfindung sind, haben einen Proteingehalt von mindestens 50% (N x 5,6) und eine weitere Zusammensetzung sowie funktionelle Eigenschaften, wie in nachfolgender Tabelle angegeben. Die so erhaltenen Sonnenblumenproteinkonzentrate (Nr.6-8) sind frei von sonnenblumeneigenen Aromakomponenten. Das Mehl (Nr. 2) wies noch einen gewissen sonnenblumennussigen Eigengeschmack auf. Nach einfacher Vermahlung und Siebung (<263 mm) wurde es zur Emulgierung einer Ei-freien Salatmayonnaise eingesetzt, die vergleichbar homogen und stabil war wie mit einem Pflanzenproteinisolat und sensorisch gut beurteilt wurde.

Die Farbe des schalenfreien Sonnenblumenproteinmehls und der Sonnenblumenproteinkonzentrate ist besonders ansprechend, d. h. neutral und weist nach CIE-L*a*b* folgende Werte auf:

**Tabelle 3-2**

| **Nr.** | **Probenbezeichnung und Herstellung** | **L*** | **a*** | **b*** |
|---|---|---|---|---|
| | **dieselben Präparate aus Tabelle 3-1** | % | % | % |
| 1 | Presskuchen | 70,5 | 1,95 | 12,75 |
| 2 | Sonnenblumenkernmehl Hexan-entölt <60°C (aus 1) | 89,21 | 0,59 | 6,48 |
| 3 | Sonnenblumenkernmehl scCO2-extrahiert 50°C, 285*10⁵ Pa (aus 1) | 88,4 | 0,49 | 7,49 |
| 4 | Sonnenblumenkernmehl scCO2-extrahiert 50°C,800*10⁵ Pa (aus 1) | 89,3 | 0,31 | 6,79 |
| 5 | Sonnenblumenkernproteinkonzentrat mit Ethanol extrahiert (aus 2) | 88,0 | -0,0 | +7,8 |

### Beispiel 4: Sonnenblumenkernproteinmehle aus entölten, geschälten Sonnenblumenkernen mit modifizierten Eigenschaften durch Einstellung der Korngröße

Bei diesem Beispiel wurde unter anderem die Modifizierung der funktionellen Eigenschaften des Sonnenblumenproteinpräparats untersucht bei einer abschließenden Aufbereitung in der Korngröße.

Herstellung:
1. Schälung der Sonnenblumensamen und Auftrennung in eine Kern- und eine Schalenfraktion
2. mechanische Teilentölung auf einen Restfettgehalt von ca. 36% durch Pressung, siehe Beispiel 2
3. Entölung des Sonnenblumenpresskuchens mit iso-Hexan in einem Perkolator bei Temperaturen von maximal 60°C
4. Austreiben des Hexans mit überhitztem Hexandampf unter Vakuum (< 500 hPa)
5. Austreiben weiteren Hexans mit überhitztem Wasserdampf unter Vakuum (< 500 hPa).
6. Entfernung von Lösemittelresten durch Erhitzung auf 60 °C im Vakuum (< 500 hPa). Das so erhaltene Raffinat wird nachfolgend Proteinmehl genannt.
7. Sichtung, Siebung und/oder Vermahlung des Proteinkonzentrats in einer Stift- oder Schlagmühle, um Fraktionen mit unterschiedlicher Partikelgrößenverteilung zu erhalten und so die funktionellen Eigenschaften zu modifizieren
8. Einsatz der Proteinmehle und Proteinkonzentrate mit oder ohne vorherige oder nachfolgende Zerkleinerung.

Das entölte Mehl (Nr. 2) wurde nur gesiebt (< 263 mm) und direkt zur Emulgierung einer Ei-freien Salatmayonnaise eingesetzt, die vergleichbar homogen und stabil war wie mit einem Pflanzenproteinisolat. Der Geschmack und die Textur konnten weiter verbessert werden, wenn das Proteinmehl vermahlen wurde.

Durch eine Aufbereitung in der Korngrösse, wie sie oben im letzten Schritt 7 durchgeführt wurde, waren die funktionellen Eigenschaften der Sonnenblumenkernproteinpräparate veränderbar. Zur Korngrössenverkleinerung wurde neben einer reinen Vermahlung auch eine Sichtung oder eine Siebung, gegebenenfalls in Verbindung mit einer Vermahlung eingesetzt. Mit abnehmender Korngrösse nahm die Wasserbindung tendenziell zu, beim SBK ebenso die Emulgierkapazität, während die Ölbindung geringfügig abnahm oder sich kaum änderte. Die Präparate mit einer homogeneren Korngrössenverteilung haben eine höhere Wasserbindung. Als besonders vorteilhaft zur Steigerung der Wasserbindung stellte sich die Kombination von Fraktionierung und Zerkleinerung heraus. Insgesamt ist es möglich, das funktionelle Profil durch eine zielgerichtete Aufbereitung in der Korngrößenverteilung zu modifizieren.

## Patentansprüche

1. Proteinpräparat, welches aus geschälten Sonnenblumensamen mit einem Restschalengehalt von ≤ 5 Gew.% hergestellt ist, die zunächst einer mechanischen Teilentölung durch Pressen bis auf einen Fett- oder Ölgehalt der geschälten Sonnenblumensamen im Bereich zwischen 10 und 35 Gew.% unterzogen wurden, und nach der weiteren Durchführung eines oder mehrerer Extraktionsschritte mit mindestens einem Lösungsmittel erhalten wird, mit einem
- Proteingehalt von mindestens 50% und weniger als 90 % bezogen auf die Trockenmasse, und
- einem Fettgehalt unter 5% bezogen auf die Trockenmasse, bestimmt nach der Büchi-Methode nach Caviezel,
- wobei das Proteinpräparat eine Helligkeit L*, bestimmt gemäß CIE-L*a*b*-Farbmessung, von mindestens 80, Werte für a* und b* gemäß CIE-L*a*b*-Farbmessung im Bereich von
- 5 < a* < +5, -5 < b* < +20 und zumindest wasserbindende, ölbindende und emulgierende Funktionalität aufweist,
- wobei die Wasserbindung des Proteinpräparats, bestimmt nach Methode 56-20 des AACC-Bestimmungsverfahrens, mindestens 2 ml/g Trockenmasse beträgt,
- die Emulgierkapazität, bestimmt nach der in der Beschreibung angegebenen Konduktivitätsmessungs-Methode nach Wäsche et al., mindestens 400 ml Öl pro Gramm beträgt, und
- die Ölbindung, bestimmt nach dem in der Beschreibung angegebenen Fettbinde-Bestimmungsverfahren aus Ludwig et al., mindestens 1 ml/g beträgt,
- und wobei das Proteinpräparat eine Proteinlöslichkeit aufweist, die, bestimmt nach der in der Beschreibung angegebenen Methode nach Morr, zwischen 30% und 60% beträgt.

2. Proteinpräparat nach Anspruch 1,
wobei die Helligkeit L* mindestens 85 und besonders bevorzugt mindestens 90, und/oder die Werte für a* und b* gemäss CIE-L*a*b*-Farbmessung im Bereich von -3 < a* < +3, -2 < b* < +15 liegen, besonders bevorzugt im Bereich von -1 < a* < +1, 0 < b* < +10.

3. Proteinpräparat nach Anspruch 1 oder 2,
wobei die Wasserbindung des Proteinpräparats, bestimmt nach Methode 56-20 des AACC-Bestimmungsverfahrens, mindestens 3 ml/g Trockenmasse beträgt und/oder die Ölbindung, bestimmt nach dem in der Beschreibung angegebenen Fettbinde-Bestimmungsverfahren aus Ludwig et al., mindestens 4 ml/g beträgt und/oder die Emulgierkapazität, bestimmt nach der in der Beschreibung angegebenen Konduktivitätsmessungs-Methode nach Wäsche et al., mindestens 500 ml Öl pro Gramm beträgt.

4. Proteinpräparat nach einem der Ansprüche 1 bis 3, wobei das Proteinpräparat eine Proteinlöslichkeit aufweist, die, bestimmt nach der in der Beschreibung angegebenen Methode nach Morr, größer als 40 % ist, bevorzugt größer als 50 %.

5. Proteinpräparat nach einem der Ansprüche 1 bis 4, wobei das Proteinpräparat mindestens eine der folgenden schaumbildenden Eigenschaften aufweist:
die Schaumaktivität entspricht mindestens 50 % der Schaumaktivität von Hühnereiklar,
die Schaumdichte entspricht mindestens 50 % und/oder höchstens 200 % der Schaumdichte von Hühnereischnee,
die Schaumstabilität entspricht mindestens 80 %, bevorzugt mindestens 90 % und besonders bevorzugt mindestens 100 % der Schaumstabilität von Hühnereischnee, bestimmt nach Aufschlag in einer Hobart-Küchenmaschine, wie zuvor beschrieben.

6. Proteinpräparat nach einem der Ansprüche 1 bis 5, wobei das Proteinpräparat einen Rohfasergehalt aufweist, der, bezogen auf die Trockenmasse, mindestens 5 %, bevorzugt mindestens 10 %, beträgt und/oder höchstens 40 %, bevorzugt höchstens 30 %.

7. Proteinpräparat nach einem der Ansprüche 1 bis 5, wobei das Proteinpräparat einen Fettgehalt aufweist, der, bezogen auf die Trockenmasse, unter 2 % liegt, besonders bevorzugt unter 1 %, bestimmt nach der Büchi-Methode nach Caviezel.

8. Proteinpräparat nach einem der Ansprüche 1 bis 7, wobei das Proteinpräparat mindestens einen Gehalt, bevorzugt alle der folgenden Gehalte an Stoffen aufweist:
Phytinsäuregehalt, bezogen auf die Trockenmasse, von unter 10 %, bevorzugt unter 2 %, besonders bevorzugt unter 1 %,
Oligosaccharidgehalt, bezogen auf die Trockenmasse, von unter 10 %, bevorzugt unter 5 %, besonders bevorzugt unter 2 %,
Phenolsäuregehalt, bezogen auf die Trockenmasse, von unter 8 %, bevorzugt unter 3 %, besonders bevorzugt unter 0,5 %.

9. Verwendung des Präparats nach einem der Ansprüche 1 bis 8 als Zutat in Futter- und Lebensmitteln, insbesondere als technofunktionelle Zutat zum Zweck der Emulsions- oder Schaumstabilisierung oder zur Texturverbesserung.

## Claims

1. Protein preparation produced from dehulled sunflower kernels with a residual kernel content of ≤ 5 wt.%, which have initially undergone a mechanical partial deoiling by pressing until a fat or oil content of the dehulled sunflower kernels is in the range between 10 and 35 wt.%, and is obtained following the further performance of one or more extraction steps with at least one solvent, with a
- protein content of at least 50% and less than 90% relative to the dry mass, and
- a fat content below 5% relative to the dry mass, determined by the Büchi method according to Caviezel,
- wherein the protein preparation has a lightness L* of at least 80 determined in accordance with CIE-L*a*b* colour measurement, values for a* and b* in accordance with CIE-L*a*b* colour measurement in the range -5 < a* < +5, -5 < b* < +20, and at least water binding, oil binding and emulsifying functional properties,
- wherein the water binding of the protein preparation determined according to method 56-20 of the AACC determination procedure, is at least 2 ml/g dry mass,
- the emulsifying capacity determined in accordance with the conductivity measurement method according to Wäsche et al. cited in the description is at least 400 ml oil per gram, and
- the oil binding determined in accordance with the fat binding determination method from Ludwig et al. cited in the description is at least 1 ml/g,
- and wherein the protein preparation exhibits a protein solubility which is between 30% and 60% as determined in accordance with the method according to Morr cited in the description.

2. Protein preparation according to Claim 1,
wherein the lightness L* is at least 85 and particularly preferably at least 90, and/or the values for a* and b* in accordance with CIE-L*a*b* colour measurement are in the range -3 < a* < +3, -2 < b* < +15, particularly preferably in the range -1 < a* < +1, 0 < b* < +10.

3. Protein preparation according to Claim 1 or 2,
wherein the water binding property of the protein preparation determined in accordance with method 56-20 of the AACC determination procedure, is at least 3 ml/g dry mass, and/or the oil binding property determined in accordance with the fat binding determination method from Ludwig et al. cited in the description is at least 4 ml/g, and/or the emulsifying capacity determined in accordance with the conductivity measurement method according to Wäsche et al. cited in the description is at least 500 ml oil per gram.

4. Protein preparation according to any of Claims 1 to 3,
wherein the protein preparation exhibits a protein solubility which is greater than 40%, preferably greater than 50% as determined in accordance with the method according to Morr cited in the description.

5. Protein preparation according to any of Claims 1 to 4,
wherein the protein preparation has at least one of the following foam-forming properties:
the foaming activity is equivalent to at least 50% of the foaming activity of chicken egg white,
the foam density corresponds to at least 50% and/or at most 200% of the foam density of beaten chicken egg white,
the foam stability is equivalent to at least 80%, preferably at least 90% and particularly preferably at least 100% of the foam stability of beaten chicken egg white, determined after whisking in a Hobart food processor as described previously.

6. Protein preparation according to any of Claims 1 to 5,
wherein the protein preparation has a raw fibre content which is at least 5%, preferably at least 10% and/or at most 40%, preferably at most 30% relative to the dry mass.

7. Protein preparation according to any of Claims 1 to 5,
wherein the protein preparation has a fat content which is below 2%, particularly preferably below 1% relative to the dry mass as determined using the Büchi method in accordance with Caviezel.

8. Protein preparation according to any of Claims 1 to 7,
wherein the protein preparation has at least one content, preferably all the following substance contents:
phytic acid content of below 10%, preferably below 2%, particularly preferably below 1%, relative to the dry mass,
oligosaccharide content of below 10%, preferably below 5%, particularly preferably below 2%, relative to the dry mass,
phenolic acid content of below 8%, preferably below 3%, particularly preferably below 0.5%, relative to the dry mass.

9. Use of the preparation according to any one of Claims 1 to 8, as an ingredient in animal feed and food products, in particular as a technofunctional ingredient for the purpose of emulsion or foam stabilisation or for improving texture.

## Revendications

1. Préparation protéique, qui est produite à partie de graines de tournesol pelées avec une teneur résiduelle en pelure de ≤ 5% en poids, qui est ensuite soumise à une élimination mécanique partielle de l'huile par compression jusqu'à une teneur en graisse ou en huile des graines de tournesol pelées comprise entre 10 et 35% en poids, et est obtenue après l'exécution d'une ou plusieurs étapes d'extraction avec au moins un solvant, avec
- une teneur en protéines d'au moins 50% et inférieure à 90% rapporté à la matière sèche et
- une teneur en matières grasses inférieure à 5% rapporté à la matière sèche, déterminée selon le procédé Büchi selon Caviezel,
- dans lequel la préparation protéique présente des valeurs de luminosité L* déterminées selon une mesure de couleur CIE-L*a*b*, d'au moins 80, pour a* et b* selon une mesure de couleur CIE-L*a*b* dans la plage de -5 < a* < +5, -5 < b* < +20 et au moins une fonctionnalité liant l'eau, liant l'huile et émulsionnante,
- dans lequel la liaison à l'eau de la préparation protéique, déterminée selon le procédé 56-20 du procédé de détermination de l'AACC, s'élève au moins à 2 ml/g de la matière sèche,
- la capacité émulsifiante, déterminée selon le procédé de mesure de la conductivité spécifié dans la description selon Wäsche et al., s'élève au moins à 400 ml d'huile par gramme, et
- la liaison à l'huile, déterminée selon le procédé de liaison aux graisses spécifié dans la description de Ludwig et al., s'élève à au moins 1 ml/g,
- et dans lequel la préparation protéique présente une solubilité protéique qui, déterminée selon le procédé Morr spécifié dans la description, est comprise entre 30% et 60%.

2. Préparation protéique selon la revendication 1,
dans laquelle la luminosité L* est d'au moins 85 et de manière particulièrement préférée d'au moins 90, et/ou les valeurs de a* et b* selon la mesure de couleur CIE-L*a*b* se situent dans la plage de -3 < a* < +3, -2 < b* < +15, de manière particulièrement préférée dans la plage de -1 < a* < +1, 0 < b* < +10.

3. Préparation protéique selon la revendication 1 ou 2,
dans laquelle la liaison à l'eau de la préparation protéique, déterminée selon le procédé de détermination 56-20 de l'AACC, s'élève au moins à 3 ml/g de matière sèche et/ou la liaison à l'huile, déterminée selon le procédé de liaison aux graisses de Ludwig et al. est d'au moins 4 ml/g et/ou le pouvoir émulsifiant, déterminé selon le procédé de mesure de la conductivité selon Wäsche et al. spécifié dans la description, est d'au moins 500 ml d'huile par gramme.

4. Préparation protéique selon une des revendications 1 à 3,
dans laquelle la préparation protéique présente une solubilité protéique qui, déterminée selon le procédé de Morr spécifié dans la description, est supérieure à 40%, de préférence supérieure à 50%.

5. Préparation protéique selon une des revendications 1 à 4,
dans laquelle la préparation protéique présente au moins une des propriétés moussantes suivantes :
l'activité de mousse correspond à au moins 50% de l'activité de mousse de l'œuf de poule clair,
la densité de mousse correspond à au moins 50% et/ou au plus 200% de la densité de mousse de la neige d'œuf de poule,
la stabilité de la mousse correspond à au moins 80%, de préférence au moins 90% et de manière particulièrement préférée au moins 100% de la stabilité de la mousse de neige d'œuf de poule, déterminée après avoir servi dans un robot culinaire Hobart, comme décrit précédemment.

6. Préparation protéique selon une des revendications 1 à 5,
dans laquelle la préparation protéique a une teneur en fibres brutes qui, sur la base de la matière sèche, est d'au moins 5%, de préférence d'au moins 10% et/ou d'au plus 40%, de préférence d'au plus 30%.

7. Préparation protéique selon une quelconque des revendications 1 à 5,
dans laquelle la préparation protéique a une teneur en matières grasses qui, par rapport à la matière sèche, est inférieure à 2%, de manière particulièrement préférée inférieure à 1%, déterminée selon le procédé de Büchi selon Caviezel.

8. Préparation protéique selon une des revendications 1 à 7,
dans laquelle la préparation protéique présente au moins une teneur, de préférence la totalité des teneurs suivantes en substances :
teneur en acide phytique, rapportée à la matière sèche, inférieure à 10%, de préférence inférieure à 2%, de manière particulièrement préférée inférieure à 1%,
teneur en oligosaccharides, rapportée à la matière sèche, inférieure à 10%, de préférence inférieure à 5%, de manière particulièrement préférée inférieure à 2%,
teneur en acide phénolique, rapportée à la matière sèche, inférieure à 8%, de préférence inférieure à 3%, de manière particulièrement préférée inférieure à 0,5%.

9. Utilisation de la préparation selon une des revendications 1 à 8 comme ingrédient dans les aliments pour animaux et les denrées alimentaires, en particulier comme ingrédient technofonctionnel à des fins de stabilisation d'émulsion ou de mousse ou d'amélioration de texture.
